# EUROPÄISCHE PATENTSCHRIFT

(11) **EP 2 436 305 B1**
(45) Veröffentlichungstag und Bekanntmachung des Hinweises auf die Patenterteilung: **05.08.2020**
(21) Anmeldenummer: 11177792.6
(22) Anmeldetag: 17.08.2011
(51) Int. Cl.: A61B 3/08, A61B 3/032

(54) **Sehtestgerät**
Eye test device
Appareil de test visuel

(30) Priorität: 30.09.2010 DE 202010013741 U
(43) Veröffentlichungstag der Anmeldung: 04.04.2012
(73) Patentinhaber: Oculus Optikgeräte GmbH, 35582 Wetzlar-Dutenhofen (DE)
(72) Erfinder: Kirchhübel, Rainer, D-35614 Asslar (DE); Feiertag, Carsten, D-35410 Hungen (DE)
(74) Vertreter: advotec.

(56) Entgegenhaltungen:
- EP-A1- 0 487 073
- CN-A- 101 556 402
- DE-A1- 10 310 589
- DE-A1- 19 501 415
- JP-A- 2003 180 633

## Beschreibung

Die Erfindung betrifft ein Sehtestgerät mit einer Abbildungseinrichtung zur virtuellen Abbildung eines innerhalb einer Brennweite der Abbildungseinrichtung befindlichen und in unterschiedlicher Entfernung abbildbaren Testobjektes in ein in einem Brennpunkt der Abbildungseinrichtung befindliches Auge eines Probanden, mit einer optischen Umlenkeinrichtung, wobei die Umlenkeinrichtung in einem Strahlengang zwischen der Abbildungseinrichtung und dem Testobjekts angeordnet ist, und den Strahlengang um 180° umlenkt, wobei die Umlenkeinrichtung in Richtung des Strahlengangs verschiebbar ausgebildet ist, und wobei das Testobjekt unverschiebbar ausgebildet ist, mit einer Monitoreinrichtung, wobei die Monitoreinrichtung zur Erzeugung des Testobjekts dient, wobei die Monitoreinrichtung alleine einen Monitor aufweist, wobei die im Strahlengang liegenden optischen Bauelemente so groß ausgebildet sind, dass beide Augen des Probanden an dem Test beteiligbar sind, und wobei so für jeweils ein Auge ein Teilstrahlengang ausbildbar ist.

Derartige Sehtestgeräte sind aus dem Stand der Technik hinreichend bekannt und dienen im Wesentlichen zur Durchführung verschiedener Sehtests an einem Probanden. Insbesondere eine Trennung des Strahlengangs in Teilstrahlengänge für jeweils ein Auge ermöglicht eine Durchführung eines binokularen Sehtests, beispielsweise zur Prüfung des stereoskopischen Sehens. Dazu wird dem Probanden für jeden Teilstrahlengang ein unterschiedliches Testobjekt dargeboten. Dies kann dadurch erreicht werden, dass für jeden Teilstrahlengang jeweils ein Monitor zur Darstellung des Testobjekts verwendet wird. Es ist jedoch nachteilig, dass die zwei Monitore in einem Abstand relativ zueinander auf einen Pupillenabstand des Probanden ausgerichtet sein müssen. Da Pupillenabstände unterschiedlicher Probanden voneinander abweichen können, wird in diesem Fall ein Sehtestergebnis ungenau.

Auch sind Sehtestanordnungen bekannt, die aus einem Monitor bzw. Bildschirmgerät und einer von einem Probanden getragenen Testbrille gebildet sind. Das Bildschirmgerät bzw. die davon dargestellten Testzeichen sowie die jeweiligen, den Augen zugeordnete Gläser der Testbrille sind unterschiedlich polarisiert, so dass der Proband mit jedem Auge ein anderes Testobjekt sieht. Nachteilig ist hier, dass ein Aufbau dieses Sehtestgerätes relativ viel Raum durch einen notwendigen Abstand zum Bildschirmgerät benötigt, und das keine verlässlichen Sehtests für verschiedene Sehabstände ohne Weiteres schnell durchzuführen sind. Auch ist es nicht möglich, beide Augen getrennt voneinander objektiv zu untersuchen, da der Proband immer bemerkt, welches seiner Augen durch die zu untersuchende Person abgedeckt wird. Ein Proband kann so sehr leicht auch eine Fehlsichtigkeit simulieren, ohne dass die zu untersuchende Person dies bemerkt.

Aus der DE 195 01 415 A1 ist ein Sehtestgerät mit den Merkmalen des Oberbegriffs des Anspruchs 1 bekannt. Das Sehtestgerät weist mechanische Verschlusselemente bzw. Blenden oder Filter auf, die in einen Strahlengang für beide Augen eingeschwenkt werden können.

Die EP 0 487 073 A1 offenbart ein Sehtestgerät mit zwei LCD-Verschlusselementen, die zwei voneinander getrennte Strahlengänge öffnen bzw. verschließen können. Weiter weist das Sehtestgerät zwei voneinander getrennte Testobjekte auf. Es ist ein synchronisiertes Öffnen und Schließen der Strahlengänge bzw. der LCD-Verschlusselemente möglich.

Ein weiteres Sehtestgerät beschreibt die DE 103 10 589 B4, bei dem ein Testobjekt aus einem LCD-Monitor gebildet ist, der entlang eines Strahlengangs des Sehtestgerätes verschiebbar angeordnet ist.

Die CN 101 556 402 A betrifft ein LED-Beleuchtungsmodul zur Hintergrundbeleuchtung bei dem mit einer Mehrzahl verschiedenfarbiger LED's weißes Licht erzeugt wird.

Die JP 2001386394A zeigt ein Sehtestgerät mit auf einem Monitor und mittels einer Scheibe darstellbaren Testobjekten sowie zwei LCD-Verschlusselemente, die zwei voneinander getrennte Strahlengänge öffnen bzw. verschließen können. Die LCD-Verschlusselemente sind mit dem Monitor synchronisiert.

Der vorliegenden Erfindung liegt daher die Aufgabe zugrunde, ein Sehtestgerät vorzuschlagen, welches kompakt und leicht handhabbar aufgebaut ist, und mit dem eine möglichst große Anzahl unterschiedlicher Sehtests ohne die vorgenannten Einschränkungen durchführbar sind.

Diese Aufgabe wird durch ein Sehtestgerät mit den Merkmalen des Anspruchs 1 und ein Verfahren mit den Merkmalen des Anspruchs 17 gelöst.

Das erfindungsgemäße Sehtestgerät umfasst eine Abbildungseinrichtung zur virtuellen Abbildung eines innerhalb einer Brennweite der Abbildungseinrichtung befindlichen und in unterschiedlicher Entfernung abbildbaren Testobjektes in ein in einem Brennpunkt der Abbildungseinrichtung befindliches Auge eines Probanden, eine optische Umlenkeinrichtung, wobei die Umlenkeinrichtung in einem Strahlengang zwischen der Abbildungseinrichtung und dem Testobjekt angeordnet ist, und den Strahlengang um 180° umlenkt, wobei die Umlenkeinrichtung in Richtung des Strahlengangs verschiebbar ausgebildet ist, und wobei das Testobjekt unverschiebbar ausgebildet ist, eine Monitoreinrichtung, wobei die Monitoreinrichtung zur Erzeugung des Testobjekts dient, wobei die Montitoreinrichtung alleine einen Monitor aufweist, wobei die im Strahlengang liegenden optischen Bauelemente so groß ausgebildet sind, dass beide Augen des Probanden an dem Test beteiligbar sind, wobei so für jeweils ein Auge ein Teilstrahlengang ausbildbar ist, und wobei eine Verschlusseinrichtung im Strahlengang angeordnet ist, wobei die Verschlusseinrichtung zwei LCD-Verschlusselemente aufweist, die jeweils den Teilstrahlengängen zugeordnet und zur zeitsynchronen Steuerung mit dem Monitor verbunden sind, wobei mittels des Monitors zwei optisch verschiedene Testobjekte gleichzeitig erkennbar darstellbar sind, wobei mittels der LCD-Verschlusselemente die jeweiligen Teilstrahlengänge zeitsynchron mit einem Wechsel einer Darstellung der Testobjekte auf dem Monitor verschließbar oder freigebbar sind, wobei die LCD-Verschlusselemente dazu eingerichtet sind, die Teilstrahlengänge unterschiedlich zu verschließen oder freizugeben, so dass nur jeweils eines der Testobjekte durch einen Teilstrahlengang sichtbar wird.

Insbesondere die Verwendung der verschiebbar ausgebildeten Umlenkeinrichtung ermöglicht eine einfache Durchführung von Sehtests mit unterschiedlichen Sehentfernungen für das Nah- und Fernsehen. Auch ist durch die Umlenkung des Strahlengangs das Sehtestgerät vergleichsweise kompakt ausbildbar, so dass es leicht transportabel und handhabbar ist. Im vorliegenden Fall ist es weiter vorgesehen alle Komponenten des Sehtestgeräts in einem Gehäuse desselben anzuordnen. Durch die Verwendung eines einzelnen Monitors für beide Teilstrahlengänge wird eine Anpassung eines Pupillenabstands nicht mehr notwendig bzw. variable Pupillenabstände von Probanden können vernachlässigt werden. Als LCD-Verschlusselemente (Liquid Crystal Display-Verschlusselemente) werden sogenannte LCD-Shutter verwendet, welche zur zeitsynchronen Steuerung mit dem Monitor verbunden sind und ein Verschließen oder Freigeben der jeweiligen Teilstrahlengänge bewirken. Dadurch wird es möglich für die jeweiligen Teilstrahlengänge mit dem einen Monitor unterschiedliche Testobjekte darzustellen, ohne dass eine Farbdarstellung der Testobjekte verwendet werden müsste. Durch ein Zusammenspiel der vorgenannten Komponenten des Sehtestgerätes können eine Reihe von weiteren Sehtests durchgeführt werden, wie beispielsweise ein Test des Farbsehens, der monokularen und binokularen Sehschärfe, des simultanen binokularen Sehens, des stereoskopischen Sehens, des Gesichtsfeldes, der Reaktion und einfacher Fehlsichtigkeiten, wie Kurzsichtigkeit, Übersichtigkeit oder Alterssichtigkeit. Insbesondere durch Verwendung des Monitors wird es erst möglich eine beliebige Anzahl von Optotypen darzustellen und dynamische Sehtests durchzuführen.

In einer Ausführungsform des Sehtestgerätes kann mittels der Verschlusseinrichtung wahlweise ein Teilstrahlengang optisch verschließbar sein. So wird es möglich mittels des Sehtestgerätes einen monokularen Sehtest durchzuführen. Ein Verschließen eines Teilstrahlenganges kann einfach dadurch erfolgen, dass das betreffende LCD-Verschlusselement vollständig abgedunkelt wird. Dies kann dadurch erreicht werden, dass das LCD-Verschlusselement zwei relativ zueinander senkrecht ausgebildete lineare Polarisationen des zugehörigen Teilstrahlengangs bewirkt.

Erfindungsgemäß sind mittels des Monitors zwei optisch verschiedene Testobjekte gleichzeitig erkennbar darstellbar, wobei mittels der LCD-Verschlusselemente bzw. den LCD-Shuttern ein Verschließen oder Freigeben der Teilstrahlengänge zeitsynchron mit einem Wechsel einer Darstellung der Testobjekte auf dem Monitor erfolgt. Alternativ können mittels des Monitors die optisch verschiedenen Testobjekte mit jeweils unterschiedlicher Polarisation gleichzeitig dargestellt werden, wobei mittels der LCD-Verschlusselemente die jeweiligen Teilstrahlengänge unterschiedlich polarisiert werden. Auch so kann ein Verschließen oder Freigeben der Teilstrahlengänge zeitsynchron mit einem Wechsel der Testobjekte auf dem Monitor erfolgen. So wird es möglich einen binokularen Sehtest durchzuführen, bei dem dem Probanden für jeden Teilstrahlengang jeweils unterschiedliche Testobjekte dargeboten werden. Die Testobjekte können von dem Monitor im Wechsel mit einer Frequenz oberhalb einer Flimmerverschmelzungsfrequenz des Auges dargestellt werden, wobei die LCD-Verschlusselemente die Teilstrahlengänge unterschiedlich Verschließen oder Freigeben, so dass nur jeweils eines der Testobjekte durch einen Teilstrahlengang sichtbar wird.

Zur Vereinfachung des Sehtestgerätes kann der Monitor ein LCD- oder ein LED-Monitor sein. Derartige Monitore weisen eine geringe Baugröße auf und sind leicht in ein Sehtestgerät an nahezu beliebiger Stelle integrierbar. Auch kann beispielsweise durch den LCD-Monitor eine gegebenenfalls erwünschte Polarisation des Strahlengangs erfolgen. Mittels des LED-Monitors kann ein bereits beleuchtetes Testobjekt dargestellt werden.

Um das Sehtestgerät kompakt zu gestalten, und an eine Höhe eines beispielsweise sitzenden Probanden gut anpassen zu können, kann die Abbildungseinrichtung ein Ablenkelement zur Ablenkung des Strahlengangs umfassen. So wird ein Aufbau des Sehtestgerätes mit einem im Wesentlichen vertikalen Strahlengang ermöglicht, welcher in Richtung einer im Wesentlichen horizontalen Blickrichtung eines Probanden umlenkbar ist. Danach steht dann zur Ausbildung des Sehtestgerätes ein Raum zwischen einer horizontalen Blickrichtung eines sitzenden Probanden und einer Tischoberfläche zur Aufstellung des Sehtestgerätes zur Verfügung.

Das Ablenkelement kann derart verstellbar ausgebildet sein, dass der in das Auge einfallende Strahlengang relativ zu einer horizontalen Sehachse des Auges um einen Winkel α neigbar ist. Da beim Fernsehen aus physiologischen Gründen die Sehachse des Auges an eine horizontale Sehachse angenähert bzw. beim Nahsehen gegenüber der horizontalen Sehachse vom Probanden unbewusst geneigt wird, kann das Sehtestgerät durch eine Neigung bzw. Verstellung des Ablenkelementes einfach an die jeweilig veränderte Sehachse angepasst werden. So kann beim Fernsehen das Ablenkelement in seiner Höhe angehoben werden, so dass eine horizontale Sehachse im Wesentlichen erreicht wird, und beim Nahsehen das Ablenkelement so weit in Richtung einer Tischoberfläche abgesenkt werden, dass die Sehachse vergleichsweise stark gegenüber der horizontalen Sehachse, in Richtung der Tischoberfläche geneigt ist.

Weiter kann eine Einblicköffnung des Sehtestgerätes relativ zu einer Aufstellfläche des Sehtestgerätes höhenverstellbar sein. Eine höhenverstellbare Einblicköffnung ist insofern vorteilhaft, wenn eine Sehachse eines Auges relativ zu einer horizontalen Sehachse geneigt werden soll, insbesondere wenn eine Sehachse in Richtung einer Tischoberfläche nach unten geneigt ist, kann die Einblicköffnung vorteilhaft in ihrer Höhe vermindert werden. Wenn bei dieser Einstellung des Sehtestgerätes ein Nahsehen des Probanden untersucht wird, kann gleichzeitig eine Länge des Strahlengangs durch Verschieben der Umlenkeinrichtung in vertikale Richtung verkürzt werden, so dass insgesamt eine Bauhöhe des Sehtestgerätes bei diesem Sehtest vorteilhaft vermindert werden kann. Die Verstellung des Ablenkelementes in einer Höhe relativ zu dem Auge des Probanden kann dann mit einer gleichzeitigen Verschwenkung bzw. Verdrehung des Ablenkelementes einhergehen, um den Strahlengang an die veränderte Sehachse anzupassen.

Besonders vorteilhaft ist es, wenn die Verschlusseinrichtung im Strahlengang zwischen dem Ablenkelement und einer Linsengruppe der Abbildungseinrichtung angeordnet ist. So ist dann für einem Probanden nicht ohne Weiteres erkennbar, welches der beiden LCD-Verschlusselemente vollständig geschlossen ist, da die LCD-Verschlusselemente nicht in der Nähe der Augen des Probanden, sondern im Inneren eines Gerätegehäuses des Sehtestgerätes angeordnet sind. Für den Probanden kann daher nicht mehr die Möglichkeit bestehen einen Sehtest bewusst zu beeinflussen bzw. einen Sehfehler zu simulieren, da er nicht ohne Weiteres erkennen kann, welches der beiden Auge gerade getestet wird.

Hinsichtlich einer Beleuchtung des Monitors ist es von Vorteil, wenn die Monitoreinrichtung eine Beleuchtungseinheit für das Testobjekt aufweist. Beispielsweise kann die Beleuchtungseinheit eine Auflichtbeleuchtung des Testobjekts ausbilden.

So kann die Beleuchtungseinheit eine LED-Lichtquelle und einen Kollimator umfassen. Eine LED-Lichtquelle ist besonders einfach miniaturisierbar und erzeugt kaum Wärmeenergie, die abgeführt werden müsste. Die LED-Lichtquelle kann eine Mehrzahl von Leuchtdioden umfassen, deren Licht durch den Kollimator auf das Testobjekt geleitet wird. Die Leuchtdioden können dabei unterschiedliche Farben bzw. Lichtfarben aufweisen, die je nach gewünschtem Sehtest variiert werden können.

Besonders vorteilhaft ist es, wenn die LED-Lichtquelle zumindest eine RGBW-LED (Rot-Grün-Blau-Weiß-LED) aufweist. Eine derartige, mit vier Chips versehene LED ermöglicht eine auf den jeweiligen Sehtest besonders angepasste Beleuchtung des Testobjekts. So können für eine Beleuchtung des Testobjekts bei einem hellen Umfeld die RGB-Chips (Rot-Grün-Blau-Chips) der LED und für eine Beleuchtung des Testobjekts bei einem mesopischen, dunklen Umfeld der Weiß-Chip verwendet werden. Die RGB-Chips verfügen über gute Farbwiedergabeeigenschaften bei hoher Lichtausbeute. Der Weiß-Chip ist hingegen zur Erzeugung niedrigerer Beleuchtungsstärken geeignet.

Die Monitoreinrichtung kann einen Strahlenteiler umfassen, der zwischen dem Monitor und der Beleuchtungseinheit im Strahlengang angeordnet ist. Der Strahlenteiler kann als Strahlenteilerwürfel mit einem halbdurchlässigen Spiegel oder als alleine ein halbdurchlässiger Spiegel ausgebildet sein. So kann der halbdurchlässige Spiegel einen Winkel von 45° relativ zu einem vertikalen Strahlengang des Sehtestgerätes aufweisen, so dass der Monitor und die Beleuchtungseinheit jeweils gegenüberliegend dem Strahlenteiler in einem horizontalen Strahlengang desselben angeordnet sein können. Dadurch wird ebenfalls ein besonders kompakter Aufbau des Sehtestgerätes möglich.

Weiter kann der Strahlenteiler einen Polarisator ausbilden bzw. ein polarisierender Strahlenteiler sein. So wird es unter anderem auch möglich genaue Teilerverhältnisse des Strahlenteilers einzustellen. Auch können zwei polarisierte Lichtstrahlen miteinander vereint werden, um eine höhere Leistung zu erreichen oder der Strahlengang kann für eine gewünschte Wechselwirkung mit der Verschlusseinrichtung polarisiert werden.

Um beispielsweise eine Blendung eines Auges zu simulieren, kann die Monitoreinrichtung eine Blendlichtquelle aufweisen, die zusammen mit dem Testobjekt im Auge des Probanden abbildbar ist. In der einfachsten Ausführungsform kann die Blendlichtquelle als eine Leuchtdiode ausgebildet sein, die so relativ zum Strahlengang des Sehtestgerätes angeordnet ist, dass eine Blendwirkung des Auges erzielbar ist. So ist es auch möglich die Blendlichtquelle im Bereich eines Strahlenteilers der Monitoreinrichtung anzuordnen und so ein von der Blendlichtquelle erzeugtes Blendlicht in den Strahlengang einzukoppeln.

Besonders vorteilhaft ist es, wenn die Blendlichtquelle in einer Abbildungsebene des Monitors angeordnet ist. So wird es möglich, das Blendlicht, welches durch eine LED ausgebildet sein kann, in einer Ebene mit den Sehzeichen bzw. dem Testobjekt anzuordnen.

Die Anordnung der Blendlichtquelle in der Abbildungsebene des Monitors kann unerwünschte Reflexe in Verbindung mit dem Strahlenteiler bewirken. Insofern ist es vorteilhaft, wenn die Blendlichtquelle einen Polarisator aufweist. Durch eine Polarisation des Blendlichts wird eine Reflexminderung desselben bewirkt. Wenn der Strahlenteiler ebenfalls einen Polarisator aufweist, ist es erforderlich die Blendlichtquelle mit dem Polarisator auf der Seite des Monitors relativ zum Strahlenteiler anzuordnen, da sonst die Polarisation des Strahlenteilers das polarisierte Blendlicht der Blendlichtquelle blockieren würde.

Um eine Funktion der Komponenten des Sehtestgerätes, insbesondere des Monitors und der Verschlusseinrichtung aufeinander abzustimmen, ist es vorteilhaft, wenn das Sehtestgerät eine Steuerungseinrichtung umfasst, mit der die betreffenden Komponenten steuerbar sind. So wird es beispielsweise möglich mittels der Steuerungseinrichtung eine Bildwechselrate des Monitors auf eine Wechselrate einer Polarisationsrichtung der LCD-Verschlusselemente anzupassen. Weiter kann mittels der Steuerungseinrichtung eine beispielsweise motorische Verstellung des Sehtestgerätes oder eine automatisierte Steuerung und Ausführung von verschiedenen Sehtests sowie deren Auswertung bewirkt werden.

Eine besonders hohe Abbildungsqualität des Testobjektes kann erzielt werden, wenn der Strahlenteiler, die Umlenkeinrichtung bzw. eventuelle Umlenkprismen, Linsen und eventuell vorhandene Einblickscheiben breitbandvergütet sind.

In einer weiteren Ausführungsform des Sehtestgerätes kann dieses mit einem Phoropter oder einer Messbrille ergänzt sein. Damit wird es möglich auch mit dem Phoropter oder der Messbrille Sehtests durchzuführen, wenn kein ausreichend großer Raum zur Sehzeichenprojektion, beispielsweise an eine gegenüberliegende Wand, zur Verfügung steht. Der Phoropter oder die Messbrille können fest am Sehtestgerät montiert bzw. mit diesem verbunden sein.

Nachfolgend wird eine bevorzugte Ausführungsform der Erfindung unter Bezugnahme auf die beigefügten Zeichnungen näher erläutert.

Es zeigen:
- **Fig. 1**: Eine perspektivische Ansicht von optischen Komponenten einer ersten Ausführungsform eines Sehtestgerätes während eines ersten Sehtests;
- **Fig. 2**: eine perspektivische Ansicht der Komponenten des Sehtestgerätes während eines zweiten Sehtests;
- **Fig. 3**: eine perspektivische Ansicht der Komponenten des Sehtestgerätes während eines dritten Sehtests;
- **Fig. 4**: eine perspektivische Ansicht von optischen Komponenten einer zweiten Ausführungsform eines Sehtestgerätes.

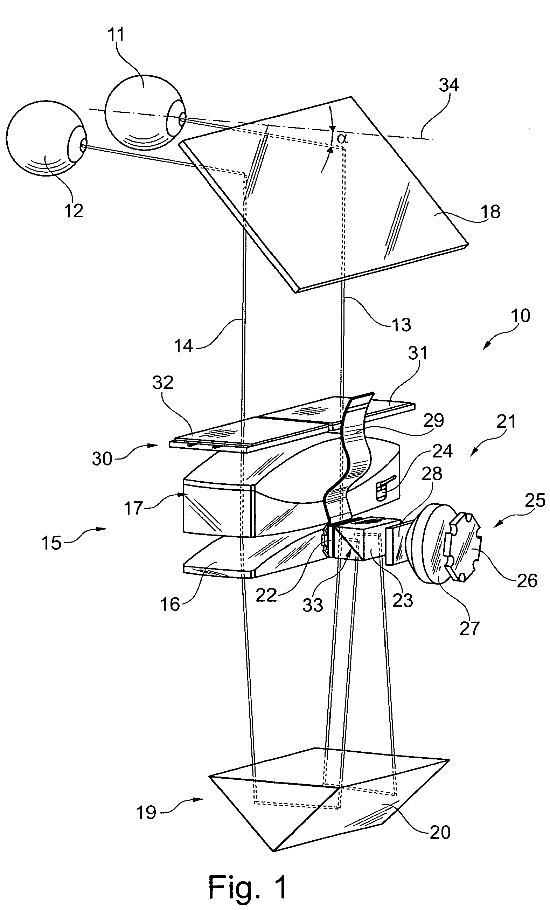

**Fig. 1** zeigt ein Sehtestgerät 10 bzw. dessen optisch wirksame Komponenten, welche in einem hier nicht sichtbar dargstellten Gehäuse des Sehtestgerätes 10 angeordnet sind. Weiter sind ein linkes Auges 11 und ein rechtes Auge 12 eines hier ebenfalls nicht gezeigten Probanden mit zugehörigen Teilstrahlengängen 13 bzw. 14 dargestellt. Das Sehtestgerät 10 umfasst eine Abbildungseinrichtung 15, gebildet aus einer Plankonvexlinse 16 und einem Achromat 17 sowie einen schwenkbaren Umlenkspiegel 18. Weiter umfasst das Sehtestgerät 10 eine Umlenkeinrichtung 19, die ein in vertikaler Richtung verschiebbares Umlenkprisma 20 aufweist. Durch eine Verschiebung des Umlenkprismas 20 wird es möglich, die Teilstrahlengänge 13 und 14 zu verkürzen bzw. deren Länge einzustellen. Das Umlenkprisma 20 ist so ausgebildet, dass die Teilstrahlengänge 13 und 14 um 180° umgelenkt werden. Darüber hinaus weist das Sehtestgerät 10 eine Monitoreinrichtung 21 auf, die einen LCD-Monitor 22, einen Strahlenteilerwürfel 23, eine Blendlichtquelle 24 und eine Beleuchtungseinheit 25 umfasst. Die Beleuchtungseinheit 25 ist wiederum aus einer LED-Lichtquelle 26, einem Kollimator 27 und einer Streuscheibe 28 gebildet. Weiter ist eine Anschlussleitung 29 zum Anschluss des LCD-Monitors 22 an eine hier nicht dargestellte Steuerungseinrichtung abgebildet. Zwischen dem Umlenkspiegel 18 und dem Achromat 17 ist eine Verschlusseinrichtung 30 angeordnet, welche aus zwei LCD-Verschlusselementen 31 und 32 für jeweils den Teilstrahlengang 13 bzw. 14 gebildet ist.

Mit Ausnahme der Verschlusseinrichtung 30 sind alle vorgenannten optischen Bauelemente so groß ausgebildet, dass beide Augen 11 und 12 des Probanden gleichzeitig auf den LCD-Monitor 22 blicken können bzw. ein darauf abgebildetes, hier nicht näher dargestelltes Testobjekt in den Augen 11 und 12 des Probanden jeweils abgebildet werden kann. Das vom LCD-Monitor 22 dargestellte Testobjekt wird mittels der Beleuchtungseinheit 25 mit Auflicht beleuchtet und über einen Umlenkspiegel 33 des Strahlenteilerwürfels 23, durch das Umlenkprisma 20, sowie die Plankonvexlinse 16 und den Achromat 17 hindurch auf den Umlenkspiegel 18 und von diesem in jeweils die Augen 11 und 12 projiziert.

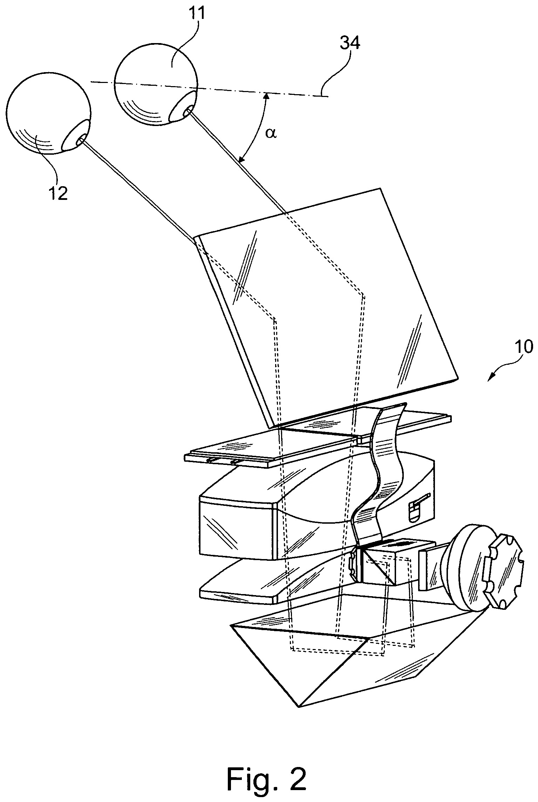

Durch eine Verschiebung des Umlenkprismas 20 in Richtung der Plankonvexlinse 16 sowie durch eine Verschiebung und Drehung des Umlenkspiegels 18 in Richtung des Achromats 17 werden die Teilstrahlengänge 13 und 14, wie in **Fig. 2** dargestellt, wesentlich verkürzt. Demnach zeigt die in **Fig. 1** dargestellte Anordnung des Sehtestgerätes 10 eine Testkonfiguration zur Untersuchung eines Fernsehens des Probanden und die in **Fig. 2** dargestellte Testkonfiguration des Sehtestgerätes 10 eine Untersuchung eines Nahsehens des Probanden. Die von den Augen 11 und 12 jeweils ausgehenden Teilstrahlengänge 13 bzw. 14 sind dabei um einen Winkel α gegenüber einer horizontalen Sehachse 34 des Probanden geneigt. Insbesondere bei der Testkonfiguration für das Nahsehen ist der Umlenkspiegel 18 soweit abgesenkt, dass der Winkel α bzw. die aus den Teilstrahlengängen 13 und 14 resultierenden Sehachsen der Augen 11 bzw. 12 einer tatsächlichen, physiologischen Blickneigung der Augen 11 und 12 beim Nahsehen angenähert sind. Auch wird so eine Bauhöhe des Sehtestgerätes 10 soweit verringert, dass eine hier nicht dargestellte Einblicköffnung in das Sehtestgerät 10 in ihrer Höhe abgesenkt wirkt.

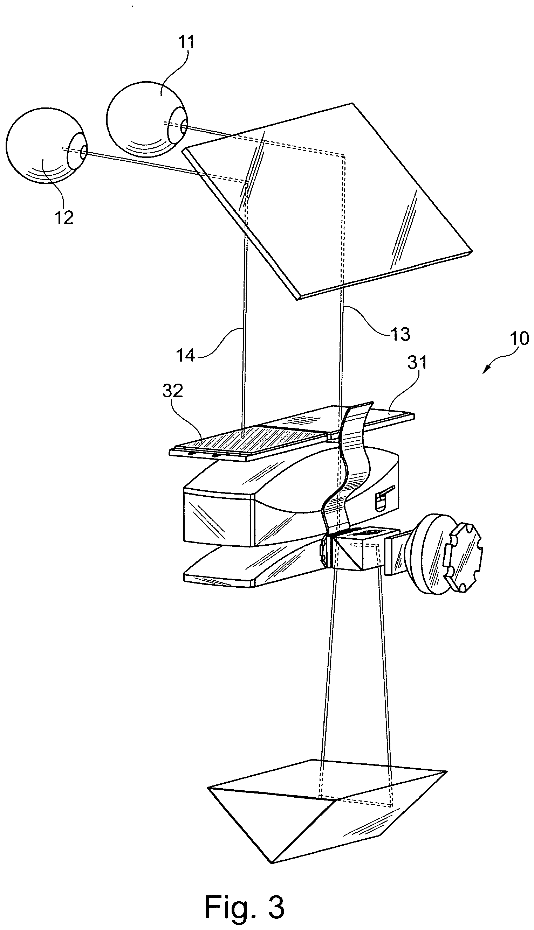

**Fig. 3** zeigt ebenfalls das Sehtestgerät 10 in einer Testkonfiguration gemäß **Fig. 1****,** wobei hier im Unterschied zur **Fig. 1** das LCD-Verschlusselement 32 abgedunkelt bzw. verschlossen ist, so dass der Teilstrahlengang 14 das LCD-Verschlusselement 32 nicht passieren kann. Somit erfolgt lediglich die Durchführung eines Sehtestes für das linke Auge 11. Da die Verschlusseinrichtung 30 innerhalb des Gehäuses des Sehtestgerätes 10 angeordnet und nicht unmittelbar für den Probanden sichtbar ist, kann der Proband nicht ohne Weiteres feststellen, welches von beiden Augen 11 oder 12 gerade getestet wird.

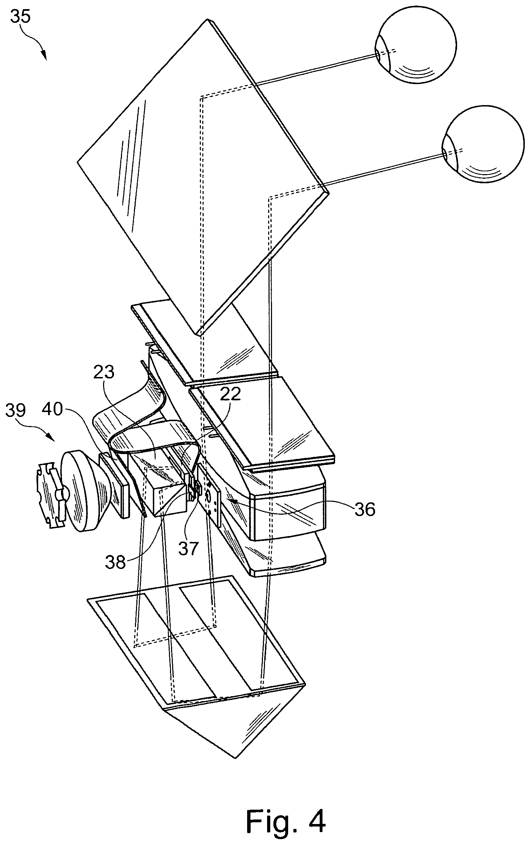

**Fig. 4** zeigt eine weitere Ausführungsform eines Sehtestgeräts 35. Das Sehtestgerät 35 entspricht im Wesentlichen dem in den **Fig. 1** bis **3** gezeigten Sehtestgerät mit dem Unterschied, dass bei dem Sehtestgerät 35 eine Blendlichtquelle 36 eine LED 37 mit einem Polarisator 38 umfasst und in einer Abbildungsebene des LCD-Monitors 22 angeordnet ist. Weiter ist bei einer Beleuchtungseinheit 39 ebenfalls ein Polarisator 40 vor dem Strahlenteilerwürfel 23 vorgesehen. So wird es möglich, eventuelle, durch die LED 37 und die Beleuchtungseinheit 39 bedingte, unerwünschte Reflexe zu vermeiden.

## Patentansprüche

1. Sehtestgerät (10, 35) mit einer Abbildungseinrichtung (15) zur virtuellen Abbildung eines innerhalb einer Brennweite der Abbildungseinrichtung befindlichen und in unterschiedlicher Entfernung abbildbaren Testobjektes in ein in einem Brennpunkt der Abbildungseinrichtung befindliches Auge (11, 12) eines Probanden, mit einer optischen Umlenkeinrichtung (19), wobei die Umlenkeinrichtung in einem Strahlengang (13, 14) zwischen der Abbildungseinrichtung und dem Testobjekt angeordnet ist, und den Strahlengang um 180 Grad umlenkt, wobei die Umlenkeinrichtung in Richtung des Strahlengangs verschiebbar ausgebildet ist, und wobei das das Testobjekt unverschiebbar ausgebildet ist, mit einer Monitoreinrichtung (21), wobei die Monitoreinrichtung zur Erzeugung des Testobjektes dient, wobei die Monitoreinrichtung alleine einen Monitor (22) aufweist, wobei die im Strahlengang liegenden optischen Bauelemente (16, 17, 18, 19, 23) so groß ausgebildet sind, dass beide Augen des Probanden an dem Test beteiligbar sind, wobei so für jeweils ein Auge ein Teilstrahlengang (13; 14) ausbildbar ist,
wobei eine Verschlusseinrichtung (30) im Strahlengang angeordnet ist, wobei die Verschlusseinrichtung zwei LCD-Verschlusselemente (31, 32) aufweist, die jeweils den Teilstrahlengängen zugeordnet und zur zeitsynchronen Steuerung mit dem Monitor verbunden sind, **dadurch gekennzeichnet, dass** mittels des Monitors zwei optisch verschiedene Testobjekte gleichzeitig erkennbar darstellbar sind, wobei mittels der LCD-Verschlusselemente die jeweiligen Teilstrahlengänge (13; 14) zeitsynchron mit einem Wechsel einer Darstellung der Testobjekte auf dem Monitor verschließbar oder freigebbar sind, wobei die LCD-Verschlusselemente dazu eingerichtet sind, die Teilstrahlengänge unterschiedlich zu verschließen oder freizugeben, so dass jeweils nur eines der Testobjekte durch einen Teilstrahlengang sichtbar wird.

2. Sehtestgerät nach Anspruch 1,
**dadurch gekennzeichnet,**
**dass** mittels der Verschlusseinrichtung (30) wahlweise ein Teilstrahlengang (13; 14) optisch verschließbar ist.

3. Sehtestgerät nach Anspruch 1 oder 2,
**dadurch gekennzeichnet,**
**dass** der Monitor (22) ein LCD- oder ein LED-Monitor ist.

4. Sehtestgerät nach einem der vorangehenden Ansprüche,
**dadurch gekennzeichnet ,**
**dass** die Abbildungseinrichtung (15) ein Ablenkelement (18) zur Ablenkung des Strahlengangs (13, 14) umfasst.

5. Sehtestgerät nach Anspruch 4,
**dadurch gekennzeichnet,**
**dass** das Ablenkelement (18) derart verstellbar ist, dass der in das Auge (11, 12) einfallende Strahlengang (13; 14) relativ zu einer horizontalen Sehachse (34) des Auges um einen Winkel α neigbar ist.

6. Sehtestgerät nach Anspruch 4 oder 5,
**dadurch gekennzeichnet,**
**dass** eine Einblicköffnung des Sehtestgerätes (10, 35) relativ zu einer Aufstellfläche des Sehtestgerätes höhenverstellbar ist.

7. Sehtestgerät nach einem der Ansprüche 4 bis 6,
**dadurch gekennzeichnet,**
**dass** die Verschlusseinrichtung (30) im Strahlengang (13; 14) zwischen dem Ablenkelement (18) und einer Linsengruppe (16, 17) der Abbildungseinrichtung (15) angeordnet ist.

8. Sehtestgerät nach einem der vorangehenden Ansprüche,
**dadurch gekennzeichnet,**
**dass** die Monitoreinrichtung (21) eine Beleuchtungseinheit (25, 39) für das Testobjekt aufweist.

9. Sehtestgerät nach Anspruch 8,
**dadurch gekennzeichnet,**
**dass** die Beleuchtungseinheit (25, 39) eine LED-Lichtquelle (26) und einen Kollimator (27) umfasst.

10. Sehtestgerät nach Anspruch 9,
**dadurch gekennzeichnet,**
**dass** die LED-Lichtquelle (26) zumindest eine RGBW-LED aufweist.

11. Sehtestgerät nach einem der Ansprüche 8 bis 10,
**dadurch gekennzeichnet,**
**dass** die Monitoreinrichtung (21) einen Strahlenteiler (23) umfasst, der zwischen dem Monitor (22)und der Beleuchtungseinheit (25, 39) im Strahlengang (13; 14) angeordnet ist.

12. Sehtestgerät nach Anspruch 11,
**dadurch gekennzeichnet,**
**dass** der Strahlenteiler (23) einen Polarisator ausbildet.

13. Sehtestgerät nach einem der vorangehenden Ansprüche,
**dadurch gekennzeichnet ,**
**dass** die Monitoreinrichtung (21) eine Blendlichtquelle (24, 36) aufweist, die zusammen mit dem Testobjekt im Auge (11, 12) des Probanden abbildbar ist.

14. Sehtestgerät nach Anspruch 13,
**dadurch gekennzeichnet,**
**dass** die Blendlichtquelle (36) in einer Abbildungsebene des Monitors (22) angeordnet ist.

15. Sehtestgerät nach Anspruch 13 oder 14,
**dadurch gekennzeichnet,**
**dass** die Blendlichtquelle (36) einen Polarisator aufweist.

16. Sehtestgerät nach einem der vorangehenden Ansprüche,
**dadurch gekennzeichnet,**
**dass** das Sehtestgerät (10, 35) eine Steuerungseinrichtung umfasst, mit der der Monitor (22) und die Verschlusseinrichtung (30) steuerbar sind.

17. Verfahren zur Durchführung eines Sehtests mit einem Sehtestgerät (10, 35), wobei das Sehtestgerät eine Abbildungseinrichtung (15), eine optische Umlenkeinrichtung (19) und eine Monitoreinrichtung (21) aufweist, wobei ein innerhalb einer Brennweite der Abbildungseinrichtung befindliches und in unterschiedlicher Entfernung abbildbares Testobjekt in ein in einem Brennpunkt der Abbildungseinrichtung befindliches Auge (11, 12) eines Probanden virtuell abgebildet wird, wobei die Umlenkeinrichtung in einem Strahlengang (13, 14) zwischen der Abbildungseinrichtung und dem Testobjekt angeordnet ist und den Strahlengang um 180 Grad umlenkt, wobei die Umlenkeinrichtung in Richtung des Strahlengangs verschiebbar ausgebildet ist, und wobei das Testobjekt unverschiebbar ausgebildet ist, wobei mittels der Monitoreinrichtung das Testobjekt erzeugt wird, wobei die Monitoreinrichtung alleine einen Monitor (22) aufweist, wobei die im Strahlengang liegenden optischen Bauelemente (16, 17, 18, 19, 23) so groß ausgebildet sind, dass beide Augen des Probanden an dem Test beteiligt sind, wobei so für jeweils ein Auge ein Teilstrahlengang (13; 14) ausgebildet wird,
wobei eine Verschlusseinrichtung (30) im Strahlengang angeordnet ist, wobei die Verschlusseinrichtung zwei LCD-Verschlusselemente (31, 32) aufweist, die jeweils den Teilstrahlengängen zugeordnet sind, wobei die LCD-Verschlusselemente und der Monitor zeitsynchron gesteuert werden, **dadurch gekennzeichnet, dass** mittels des Monitors zwei optisch verschiedene Testobjekte gleichzeitig erkennbar dargestellt werden, wobei mittels der LCD-Verschlusselemente die jeweiligen Teilstrahlengänge (13; 14) zeitsynchron mit einem Wechsel einer Darstellung der Testobjekte auf dem Monitor verschlossen oder freigegeben werden, wobei die LCD-Verschlusselemente die Teilstrahlengänge unterschiedlich verschließen oder freigeben, so dass jeweils nur eines der Testobjekte durch einen Teilstrahlengang sichtbar wird.

## Claims

1. An eyesight testing apparatus (10, 35) comprising an imaging device (15) for the virtual imaging of a test object, which is located within a focal length of the imaging device and can be imaged at different distances, into an eye (11, 12) of a subject located in a focal point of the imaging device, comprising an optical deflection device (19), the deflection device being arranged in a beam path (13, 14) between the imaging device and the test object and deflecting the beam path by 180 degrees, the deflection device being displaceable in the direction of the beam path, and the test object not being displaceable, comprising a monitor device (21), the monitor device generating the test object, the monitor device comprising a single monitor (22), the optical components (16, 17, 18, 19, 23) lying in the beam path being of such a size that both eyes of the subject are able to take part in the test, it thus being possible for a partial beam path (13; 14) to be formed for each eye, a blocking device (30) being arranged in the beam path, the blocking device comprising two LCD blocking elements (31, 32) which are each assigned to the partial beam paths and which are connected to the monitor for being controlled synchronously,
**characterised in that**
two visually different test objects can be displayed at the same time in a discernible manner by means of the monitor, the respective partial beam paths (13; 14) being blockable or releasable by means of the LCD blocking elements simultaneously with a change of a display of the test objects on the monitor, the LCD blocking elements being designed to block or release the partial beam paths separately so that in each case only one of the test objects becomes visible through a partial beam path.

2. The eyesight testing apparatus according to claim 1,
**characterised in that**
one partial beam path (13; 14) can be visually blocked selectively by means of the blocking device (30).

3. The eyesight testing apparatus according to claim 1 or 2,
**characterised in that**
the monitor (22) is an LCD or an LED monitor.

4. The eyesight testing apparatus according to any one of the preceding claims,
**characterised in that**
the imaging device (15) comprises a diverting element (18) for diverting the beam path (13, 14).

5. The eyesight testing apparatus according to claim 4,
**characterised in that**
the diverting element (18) is adjustable in such a way that the beam path (13; 14) incident in the eye (11, 12) can be inclined relative to a horizontal optical axis (34) of the eye about an angle α.

6. The eyesight testing apparatus according to either claim 4 or claim 5,
**characterised in that**
a viewing opening in the eyesight testing apparatus (10, 35) can be vertically adjusted relative to an installation surface of the eyesight testing apparatus.

7. The eyesight testing apparatus according to any one of claims 4 to 6,
**characterised in that**
the blocking device (30) is arranged in the beam path (13; 14) between the diverting element (18) and a lens group (16, 17) of the imaging device (15).

8. The eyesight testing apparatus according to any one of the preceding claims,
**characterised in that**
the monitor device (21) comprises an illumination unit (25, 39) for the test object.

9. The eyesight testing apparatus according to claim 8,
**characterised in that**
the illumination unit (25, 39) comprises an LED light source (26) and a collimator (27).

10. The eyesight testing apparatus according to claim 9,
**characterised in that**
the LED light source (26) comprises at least one RGBW LED.

11. The eyesight testing apparatus according to any one of claims 8 to 10,
**characterised in that**
the monitor device (21) comprises a beam splitter (23) which is arranged in the beam path (13; 14) between the monitor (22) and the illumination unit (25, 39).

12. The eyesight testing apparatus according to claim 11,
**characterised in that**
the beam splitter (23) forms a polariser.

13. The eyesight testing apparatus according to any one of the preceding claims,
**characterised in that**
the monitor device (21) comprises a glare source (24, 36) which can be imaged in the eye (11, 12) of the subject together with the test object.

14. The eyesight testing apparatus according to claim 13,
**characterised in that**
the glare source (36) is arranged in an imaging plane of the monitor (22).

15. The eyesight testing apparatus according to either claim 13 or claim 14,
**characterised in that**
the glare source (36) comprises a polariser.

16. The eyesight testing apparatus according to any one of the preceding claims,
**characterised in that**
the eyesight testing apparatus (10, 35) comprises a control device with which the monitor (22) and the blocking device (30) can be controlled.

17. A method for carrying out an eye test with an eyesight testing apparatus (10, 35), the eyesight testing apparatus comprising an imaging device (15), an optical deflection device (19) and a monitor device (21), a test object, which is located within a focal length of the imaging device and can be imaged at different distances, being virtually imaged into an eye (11, 12) of a subject located in a focal point of the imaging device, the deflection device being arranged in a beam path (13, 14) between the imaging device and the test object and deflecting the beam path by 180 degrees, the deflection device being displaceable in the direction of the beam path, and the test object not being displaceable, the test object being generated by means of the monitor device, the monitor device comprising a single monitor (22), the optical components (16, 17, 18, 19, 23) lying in the beam path being of such a size that both eyes of the subject take part in the test, a partial beam path (13; 14) thus being formed for each eye, a blocking device (30) being arranged in the beam path, the blocking device comprising two LCD blocking elements (31, 32) which are each assigned to the partial beam paths, the LCD blocking elements and the monitor being controlled synchronously, **characterised in that**
two visually different test objects are displayed at the same time in a discernible manner by means of the monitor, the respective partial beam paths (13; 14) being blocked or released by means of the LCD blocking elements simultaneously with a change of a display of the test objects on the monitor, the LCD blocking elements blocking or releasing the partial beam paths separately so that only one of the test objects becomes visible through a partial beam path.

## Revendications

1. Appareil d'examen de la vue (10, 35) comportant une unité d'imagerie (15) pour imager virtuellement un objet de test dans un œil (11, 12) d'une personne examinée situé dans un foyer de l'unité d'imagerie, ledit objet de test se trouvant dans une distance focale de l'unité d'imagerie et pouvant être imagé à des distances variées, comportant une unité de déviation optique (19), ladite unité de déviation étant disposée dans une trajectoire optique (13, 14) entre l'unité d'imagerie et l'objet de test et déviant la trajectoire optique de 180 degrés, ladite unité de déviation étant déplaçable dans la direction de la trajectoire optique, et ledit objet de test n'étant pas déplaçable, comportant une unité écran (21), ladite unité écran étant destinée pour générer l'objet de test, ladite unité écran présentant un seul écran (22), les composants optiques (16, 17, 18, 19, 23) situés dans la trajectoire optique étant dimensionnés tellement que les deux yeux de la personne examinée peuvent participer au test, une trajectoire optique partielle (13; 14) pouvant être formée pour chaque œil de telle façon, une unité de barrage (30) étant disposée dans la trajectoire optique, ladite unité de barrage présentant deux éléments de barrage à cristaux liquides (31, 32) qui sont chacun attribués à une des trajectoires optiques partielles et qui sont reliés avec l'écran pour être commandés de façon synchrone,
**caractérisé en ce que**
deux objets de test qui se distinguent visuellement peuvent être affichés simultanément de façon reconnaissable moyennant l'écran, lesdites trajectoires optiques partielles (13; 14) respectives pouvant être barrées ou désobstruées moyennant les éléments de barrage à cristaux liquides simultanément à un changement d'un affichage des objets de test sur l'écran, lesdits éléments de barrage étant configurés pour barrer ou désobstruer les trajectoires optiques partielles séparément de sorte qu'un seul objet de test parmi les objets de test devient visible à travers une trajectoire optique partielle chaque fois.

2. Appareil d'examen de la vue selon la revendication 1, **caractérisé en ce**
**qu'**une trajectoire optique partielle (13; 14) peut être barrée ou désobstruée visuellement au choix moyennant l'unité de barrage (30).

3. Appareil d'examen de la vue selon la revendication 1 ou 2, **caractérisé en ce que**
l'écran (22) est un écran à cristaux liquides ou un écran à DEL.

4. Appareil d'examen de la vue selon l'une quelconque des revendications précédentes,
**caractérisé en ce que**
l'unité d'imagerie (15) comprend un élément de déviation (18) pour dévier la trajectoire optique (13, 14).

5. Appareil d'examen de la vue selon la revendication 4, **caractérisé en ce que**
l'élément de déviation (18) est ajustable de telle manière que la trajectoire optique (13; 14) entrant dans l'œil (11, 12) est inclinable d'un angle α par rapport à un axe visuel horizontal (34) de l'œil.

6. Appareil d'examen de la vue selon la revendication 4 ou 5, **caractérisé en ce**
**qu'**une ouverture de vue dans l'appareil d'examen de la vue (10, 35) est réglable en hauteur par rapport à une surface de montage de l'appareil d'examen de la vue.

7. Appareil d'examen de la vue selon l'une quelconque des revendications 4 à 6,
**caractérisé en ce que**
l'unité de barrage (30) est disposée dans la trajectoire optique (13; 14) entre l'élément de déviation (18) et un groupe de lentilles (16, 17) de l'unité d'imagerie (15).

8. Appareil d'examen de la vue selon l'une quelconque des revendications précédentes,
**caractérisé en ce que**
l'unité écran (21) présente une unité d'illumination (25, 39) pour l'objet de test.

9. Appareil d'examen de la vue selon la revendication 8, **caractérisé en ce que**
l'unité d'illumination (25, 39) comprend une source lumineuse à DEL (26) et un collimateur (27).

10. Appareil d'examen de la vue selon la revendication 9,
**caractérisé en ce que**
la source lumineuse à DEL (26) présente au moins une diode électroluminescente rouge-vert-bleu-blanc.

11. Appareil d'examen de la vue selon l'une quelconque des revendications 8 à 10,
**caractérisé en ce que**
l'unité écran (21) comprend un séparateur de faisceau (23) qui est disposé dans la trajectoire optique (13; 14) entre l'écran (22) et l'unité d'illumination (25, 39).

12. Appareil d'examen de la vue selon la revendication 11, **caractérisé en ce que**
le séparateur de faisceau (23) forme un polariseur.

13. Appareil d'examen de la vue selon l'une quelconque des revendications précédentes,
**caractérisé en ce que**
l'unité écran (21) présente une source de lumière éblouissante (24, 36) qui peut être imagé dans l'œil (11, 12) de la personne examinée conjointement avec l'objet de test.

14. Appareil d'examen de la vue selon la revendication 13, **caractérisé en ce que**
la source de lumière éblouissante (36) est disposée dans un plan d'imagerie de l'écran (22).

15. Appareil d'examen de la vue selon l'une quelconque des revendications 13 ou 14,
**caractérisé en ce que**
la source de lumière éblouissante (36) présente un polariseur.

16. Appareil d'examen de la vue selon l'une quelconque des revendications précédentes,
**caractérisé en ce que**
l'appareil d'examen de la vue (10, 35) comprend une unité de commande avec laquelle l'écran (22) et l'unité de barrage (30) peuvent être commandés.

17. Procédé destiné à exécuter un examen de la vue à l'aide d'un appareil d'examen de la vue (10, 35), ledit appareil d'examen de la vue présentant une unité d'imagerie (15), une unité de déviation optique (19) et une unité écran (21), un objet de test qui se trouve dans une distance focale de l'unité d'imagerie et qui peut être imagé à des distances variées étant imagé virtuellement dans un œil (11, 12) d'une personne examinée situé dans un foyer de l'unité d'imagerie, ladite unité de déviation étant disposée dans une trajectoire optique (13, 14) entre l'unité d'imagerie et l'objet de test et déviant la trajectoire optique de 180 degrés, ladite unité de déviation étant déplaçable dans la direction de la trajectoire optique, et ledit objet de test n'étant pas déplaçable, ledit objet de test étant généré moyennant l'unité écran, ladite unité écran présentant un seul écran (22), les composants optiques (16, 17, 18, 19, 23) situés dans la trajectoire optique étant dimensionnés tellement que les deux yeux de la personne examinée peuvent participer au test, une trajectoire optique partielle (13; 14) étant formée pour chaque œil de telle façon, une unité de barrage (30) étant disposée dans la trajectoire optique, ladite unité de barrage présentant deux éléments de barrage à cristaux liquides (31, 32) qui sont chacun attribués à une des trajectoires optiques partielles, lesdits éléments de barrage à cristaux liquides et ledit écran étant commandés de façon synchrone,
**caractérisé en ce que**
deux objets de test qui se distinguent visuellement sont affichés simultanément de façon reconnaissable moyennant l'écran, lesdites trajectoires optiques partielles (13; 14) respectives étant barrées ou désobstruées moyennant les éléments de barrage à cristaux liquides simultanément à un changement d'un affichage des objets de test sur l'écran, lesdits éléments de barrage barrant ou désobstruant les trajectoires optiques partielles séparément de sorte qu'un seul objet de test parmi les objets de test devient visible à travers une trajectoire optique partielle chaque fois.
